# EUROPÄISCHE PATENTSCHRIFT

(11) **EP 0 741 123 B1**
(45) Veröffentlichungstag und Bekanntmachung des Hinweises auf die Patenterteilung: **18.09.2002**
(21) Anmeldenummer: 96106481.3
(22) Anmeldetag: 25.04.1996
(51) Int. Cl.: C07C 63/68, C07C 51/00, C07C 51/54, C07C 63/16, C07C 63/20

(54) **Verfahren zur Herstellung von Salzen substituierter oder unsubstituierter Phthalsäure-Derivate**
Process for the preparation of salts of substituted or unsubstituted phthalic acid derivatives
Procédé pour la préparation de sels de dérivés d'acide phtalique substitués ou non-substitués

(30) Priorität: 02.05.1995 DE 19515986
(43) Veröffentlichungstag der Anmeldung: 06.11.1996
(73) Patentinhaber: Clariant GmbH, 65929 Frankfurt am Main (DE)
(72) Erfinder: Pfirmann, Ralf, Dr., 64347 Griesheim (DE); Papenfuhs, Theodor, Dr., 60433 Frankfurt (DE)

(56) Entgegenhaltungen:
- EP-A- 0 259 663
- EP-A- 0 510 491
- EP-A- 0 578 165
- KEMP R. & KEEGAN S.E.: 'Calcium Chloride' ULLMANN'S ENCYCLOPEDIA OF INDUSTRIAL CHEMISTRY Bd. A4, 1985, VCH WEINHEIM, Seiten 547 - 553

## Beschreibung

Die vorliegende Erfindung betrifft ein Verfahren zur Herstellung von Salzen substituierter oder unsubstituierter, insbesondere substituierter Phthalsäuren.

Innerhalb der Gruppe der substituierten Phthalsäuren spielen halogenierte Phthalsäuren, insbesondere chlorierte und fluorierte Phthalsäuren, eine wichtige Rolle. Die chlorierten Phthalsäuren dienen als Ausgangsmaterial für fluorierte Phthalsäuren, die ihrerseits wichtige Zwischenprodukte für die Herstellung von antibakteriellen Mitteln (DOS 33 18 145, EP 424 850, EP 271 275) darstellen.

Tetrafluorphthalsäure dient zum Beispiel als Einsatzstoff für die Herstellung von Polymeren (JP 02/29406), läßt sich aber auch für die Herstellung von photosensitiven Materialien (JP 01/268662, JP 11955 (1986)) oder von Flüssigkristallen (EP 0 602 596, EP 0 602 549) verwenden.

In der Literatur sind verschiedene Wege zur Herstellung von Tetrafluorphthalsäure beschrieben.

Tetrafluorphthalsäure kann beispielsweise aus Tetrachlorphthaloylchlorid (G.G. Yakobsen et al., Zh. Obshsh. Khim. 36 (1966), 139; EP 0 140 482, GB 2 146 635), aus Tetrachloranthranilsäure (S. Hayashi et al., Bull. Chem. Soc. Jap. 45 (1972), 2909), aus 1,2,3,4-Tetrafluorbenzol (L.J. Belf et al., Tetrahedron 23 (1967), 4719; Z. Naturforsch. 31B (1976), 1667), aus Tetrachlorphthalsäureanhydrid (DE-OS 3 810 093; EP 0 218 111) oder aus Tetrachlorphthalodinitril (GB 2 134 900) über teilweise aufwendige und/oder technisch nicht oder nur schwer zu verwirklichende Schritte hergestellt werden. Dieselbe Aussage trifft auch für die Herstellung von Tetrafluorphthalsäure aus 1,2-Dibromtetrafluorbenzol (C. Tamborski et al., J. Organometallic Chem., 10 (1967), 385) und die von P. Sartori et al. (Chem. Ber. 101 (1968), 2004) beschriebene Methode, ausgehend von Octafluornaphthalin, zu. Ebenfalls werden N-kohlenstoffsubstituierte Tetrachlorphthalimide (EP 0 259 663) eingesetzt. Nach Fluorierung können diese über teilweise unselektive Schritte (JP 02/145 538) ohne Zwischenisolierung der Tetrafluorphthalsäure, jedoch unter Isolierung eines ihrer funktionellen Derivate, zu 2,3,4,5-Tetrafluorbenzoesäure, einem ebenfalls für die Synthese von antibakteriellen Mitteln wichtigen Vorprodukt, umgesetzt werden. Die zwischenisolierten funktionellen Derivate können zu Tetrafluorphthalsäure hydrolysiert werden.

Ein anderes Verfahren geht von N-substituierten Tetrafluorphthalimiden aus, die mittels saurer Hydrolyse in Tetrafluorphthalsäure überführt werden (EP 0 578 165). Diese Umsetzung kann auch ohne Zusatz saurer Katalysatoren durchgeführt werden. Ein Grund hierfür ist wahrscheinlich, daß die während der Hydrolyse abgespaltene Tetrafluorphthalsäure die Reaktion im Sinne eines autokatalysierten Prozesses ablaufen läßt. Nachteilig an diesem Verfahren ist, daß es zum einen auf die Umsetzung von Tetrafluorphthalimiden beschränkt ist und lediglich Tetrafluorphthalsäure zugänglich macht, und daß zum anderen die Umsetzung im sauren Medium durchgeführt wird, was zu erheblichen Korrosionsproblemen führt.

Die Herstellung von N-substituierten Tetrafluorphthalimiden ist in der EP 510 491 beschrieben. Man setzt Tetrachlorphthalsäureanhydrid mit mindestens der äquimolaren Menge, zweckmäßigerweise mit einem Überschuß, an Hydrazin oder einem N,N-disubstituierten Hydrazin in wäßrig alkoholischem Medium, in Eisessig, in Schwefelsäure oder Oleum bei Temperaturen von 100 bis 220°C zum entsprechenden N'-substituierten N-Aminotetrachlorphthalimid um und tauscht anschließend Chlor gegen Fluor aus (Halex-Reaktion). Diese Arbeitsweise läßt sich allgemein auf die Herstellung von N-substituierten Phthalimiden übertragen, indem man die entsprechenden substituierten oder unsubstituierten Phthalsäuren mit Hydrazin oder einem entsprechend substituierten Hydrazin umsetzt und falls gewünscht, einen Chlor-Fluor-Austausch durchführt.

Der vorliegenden Erfindung liegt die Aufgabe zugrunde, ein einfaches Verfahren zur Herstellung von Salzen substituierter oder unsubstituierter Phthalsäuren, bereitzustellen, das sich ohne großen technischen Aufwand realisieren läßt und Salze einer Vielzahl von Phthalsäuren zugänglich macht, aus denen sich durch einfaches Ansäuern die entsprechenden Phthalsäuren freisetzen lassen. Das Verfahren soll die gewünschten Wertprodukte mit hohem Umsatz und hoher Selektivität liefern und möglichst keine Probleme hinsichtlich Korrosion bereiten.

Diese Aufgabe wird gelöst durch ein Verfahren zur Herstellung von Salzen substituierter oder unsubstituierter Phthalsäuren. Es ist dadurch gekennzeichnet, daß man eine Verbindung der allgemeinen Formel 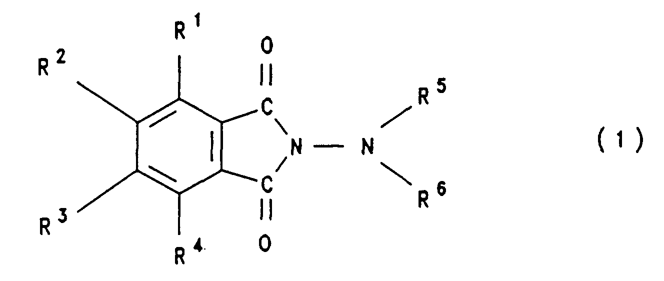 worin R¹, R², R³ und R⁴ gleich oder verschieden sind und für H, F, Cl, Br, CF₃, OH, für einen Alkoxy- oder Alkylrest mit jeweils 1 bis 4 Kohlenstoffatomen oder einen Rest -NR⁷R⁸ stehen, in welchem R⁷ und R⁸ gleich oder verschieden sind und für H, einen Alkylrest mit 1 bis 4 Kohlenstoffatomen oder einen Phenylrest stehen, R⁵ und R⁶ gleich oder verschieden sind und für H, eine -CO-Alkylgruppe mit 1 bis 6 Kohlenstoffatomen im Alkylrest oder eine Benzoylgruppe stehen, oder R⁵ und R⁶ zusammen einen Rest der allgemeinen Formel 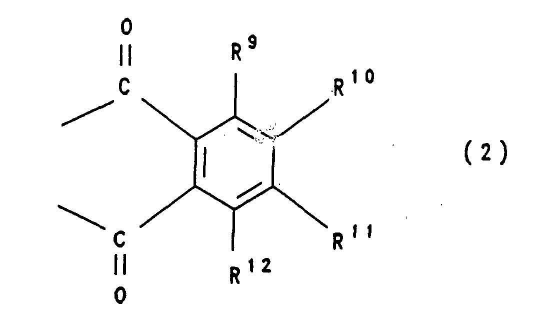 bilden, worin R⁹, R¹⁰, R¹¹ und R¹² gleich oder verschieden sind und für H, F, Cl, Br, CF₃, OH, für einen Alkoxy- oder Alkylrest mit jeweils 1 bis 4 Kohlenstoffatomen oder einen Rest -NR⁷R⁸ stehen, in welchem R⁷ und R⁸ gleich oder verschieden sind und für H, einen Alkylrest mit 1 bis 4 Kohlenstoffatomen oder einen Phenylrest stehen, mit Wasser, 2 bis 30 Äquivalenten einer in Wasser löslichen Base je Mol abzuspaltender Phthalsäuresalze und einem Oxidationsmittel bei einer Temperatur von -10 bis 150°C in Anwesenheit oder Abwesenheit eines in Wasser unlöslichen, gegenüber den Reaktionsbedingungen inerten Lösungsmittels umsetzt, wobei die in Wasser lösliche Base und das Oxidationsmittel gleichzeitig eingesetzt werden.

Das erfindungsgemäße Verfahren weist gegenüber dem Stand der Technik, insbesondere gegenüber der EP 0 578 165, mehrere Vorteile auf. Es läßt sich für die Herstellung von Salzen einer Vielzahl verschiedener Phthalsäuren anwenden und ist nicht auf den Einsatz von N-substituierten Tetrafluorphthalimiden beschränkt. Probleme bezüglich Korrosion, hervorgerufen durch Säuren bzw. Bildung von Säuren, beispielsweise HF, treten nicht auf, da die in Wasser lösliche Base das Entstehen freier Säure unterbindet. Dadurch entfallen auch die bei Verwendung eines sauren Katalysators, beispielsweise Mineralsäure, auftretenden Korrosionsprobleme. Im Vergleich zu dem Verfahren der EP 0 578 165 läßt sich das erfindungsgemäße Verfahren unter noch milderen Bedingungen, insbesondere niedrigeren Temperaturen ohne Gefahr von Korrosion durchführen und liefert darüber hinaus noch höhere Ausbeuten. Ein zusätzlicher Vorteil besteht darin, daß man die Salze in Form ihrer Lösung, wie sie als Reaktionsprodukt anfallen, ohne größeren Aufwand weiterverarbeiten kann. Eine Isolierung der entsprechenden substituierten oder unsubstituierten Phthalsäuren ist dabei nicht erforderlich. Ferner lassen sich, falls gewünscht, mit Hilfe des erfindungsgemäßen Verfahrens auch die entsprechenden Phthalsäuren auf sehr einfache Weise, nämlich durch Ansäuern der wäßrigen Phthalsäuresalzlösungen, herstellen.
Das erfindungsgemäße Verfahren läßt sich flexibel handhaben, indem man beispielsweise zunächst bei niedrigeren Temperaturen und anschließend bei relativ hohen Temperaturen oder von Anfang an bei relativ hohen Temperaturen arbeitet und außer der Hydrolyse noch eine Austauschreaktion - beispielsweise Halogen, insbesondere Fluor, gegen Hydroxid ablaufen läßt. Will man einen derartigen Austausch unterbinden, führt man das Verfahren bei niedrigeren Temperaturen durch.
Es ist als überraschend anzusehen, daß unter den Bedingungen der Reaktion Halogene, insbesondere Fluor, nicht unkontrollierbar gegen Hydroxygruppen ausgetauscht werden, sondern daß es möglich ist, diesen Austausch nach Belieben zu vermeiden oder gezielt durchzuführen.

Man kann mit gutem Erfolg eine Verbindung der Formel (1), worin R¹, R², R³ und R⁴ gleich oder verschieden sind und für H, F, Cl, OH oder für einen Alkoxyrest mit 1 bis 4 Kohlenstoffatomen, insbesondere für F, OH oder einen Alkoxyrest mit 1 bis 4 Kohlenstoffatomen stehen, in das Verfahren einsetzen.

Man setzt in das Verfahren insbesondere eine Verbindung der Formel (1) ein, worin R⁵ und R⁶ einen Rest (2) bilden, R⁹, R¹⁰, R¹¹ und R¹² gleich oder verschieden sind und für H, F, Cl, OH oder für einen Alkoxyrest mit 1 bis 4 Kohlenstoffatomen, insbesondere für F, OH oder einen Alkoxyrest mit 1 bis 4 Kohlenstoffatomen, stehen.

Von besonderem Interesse sind Verbindungen der Formel (3) 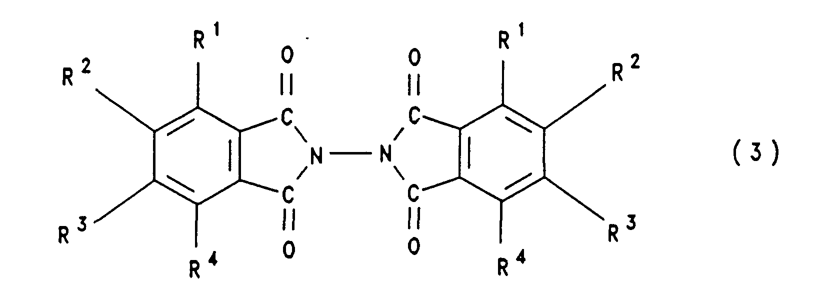 worin R¹, R², R³ und R⁴ die voranstehende Bedeutung haben. 1 Mol dieser Verbindungen liefert jeweils 2 Mol der entsprechenden Phthalsäuresalze. Zudem wird die Bildung weiterer Nebenprodukte, die aus der Hydrolyse entstammen, vermieden.

Fluorierte Verbindungen der Formel (1) sind ebenfalls von Bedeutung.

Bei diesen Verbindungen handelt es sich um N'-substituierte N-Aminofluorphthalimide, die neben Fluor auch noch andere Substituenten enthalten können. Sie lassen sich beispielsweise ausgehend von entsprechend substituierten Chlorphthalsäureanhydriden oder entsprechend substituierten Chlorphthalsäuren durch Umsetzung mit Hydrazinsulfat oder einen entsprechend substituierten Hydrazinsulfat in Schwefelsäure und nachfolgenden Chlor-Fluor-Austausch auf vergleichsweise einfache Art herstellen.

Ohne Anspruch auf Vollständigkeit zu erheben, seien als geeignete N'-substituierte N-Aminofluorphthalimide N'-Diacylaminomonofluorphthalimide, N'-Diacylaminodifluorphthalimide, N-Diacylaminotetrafluorphthalimide, 3,4,6-Trifluorbisphthalimide, Octafluorbisphthalimid, N'-Dibenzoylaminomonofluorphthalimide, N'-Dibenzoylaminodifluorphthalimide, N'-Dibenzoylaminotetrafluorphthalimide, Hexafluorbisphthalimide, Tetrafluorbisphthalimide, insbesondere Hexafluorbisphthalimid und Octafluorbisphthalimid genannt.

Eine besonders interessante Rolle kommt dem Octafluorbisphthalimid 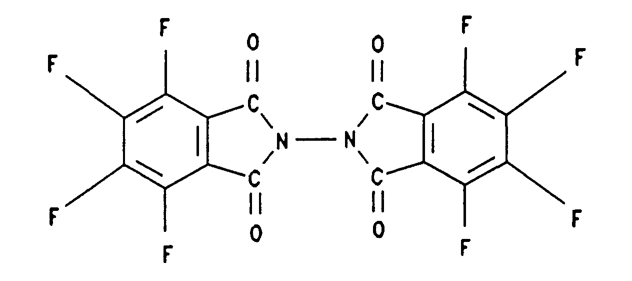 zu, das durch die vorstehende Formel wiedergegeben wird.

Das erfindungsgemäße Verfahren läßt sich nämlich besonders vorteilhaft unter Einsatz von Octafluorbisphthalimid durchführen. Bei der Hydrolyse von einem Mol Octafluorbisphthalimid entstehen 2 Mol Tetrafluorphthalsäuresalze, die gegebenenfalls durch Ansäuern in Tetrafluorphthalsäure oder Tetrafluorphthalsäureanhydrid überführt werden können. Dies gestaltet die Reaktion besonders einfach und führt auch zur Vermeidung weiterer, aus der Hydrolyse entstammender Nebenprodukte.

Zur Durchführung der Reaktion setzt man 2 bis 3000, insbesondere 10 bis 500, bevorzugt 30 bis 200 Mol Wasser je Mol abzuspaltender Phthalsäuresalze zu.

Will man lediglich die Hydrolyse durchführen, so setzt man üblicherweise vergleichsweise geringe Mengen Wasser, beispielsweise 2 bis 10, insbesondere 2,2 bis 6 Mol Wasser je Mol abzuspaltender Phthalsäure zu.

Möchte man hingegen eine wäßrige Lösung von Salzen der Phthalsäuren, wie sie zu weiteren Verarbeitung eingesetzt werden kann, herstellen, so empfiehlt es sich, vergleichsweise große Mengen Wasser, beispielsweise 50 bis 500, insbesondere 100 bis 300 Mol Wasser je Mol abzuspaltender Phthalsäuresalze zu verwenden.

Es empfiehlt sich somit generell, Wasser zumindest in der stöchiometrisch erforderlichen Menge oder vorteilhafterweise in einem entsprechend gewählten Überschuß einzusetzen. Wie groß man den Wasserüberschuß wählt hängt, wie vorstehend bereits erläutert, von der jeweilige Variante der Hydrolyse ab.

Üblicherweise setzt man - wie eingangs erwähnt - 2 bis 30, insbesondere 3 bis 15, bevorzugt 4 bis 10 Äquivalente der in Wasser löslichen Base je Mol abzuspaltender Phthalsäuresalze an.

Man kann für die Umsetzung jede Base verwenden, die stark genug ist, die gewünschte Hydrolyse herbeizuführen. Geeignete in Wasser lösliche Basen sind Alkalioxide, Alkalihydroxide, Erdalkalioxide oder Erdalkalihydroxide oder Gemische derselben. Besonders geeignet sind Natriumhydroxid oder Kaliumhydroxid.

Die Umsetzung verläuft im basischen Medium. Es kann nützlich sein, während der Hydrolyse einen bestimmten pH-Wert, beispielsweise pH = 10, insbesondere pH = 11, bevorzugt pH = 12, nicht zu unterschreiten. Falls gewünscht, kann man die in Wasser lösliche Base in dem Maße zugeben, wie sie durch die ablaufende Reaktion verbraucht wird. Auf diese Weise läßt sich die Umsetzung gezielt durchführen und einfach überwachen.

Zur Durchführung des Verfahrens wird ferner ein Oxidationsmittel benötigt. Geeignet als Oxidationsmittel ist jedes Oxidans, das unter den Bedingungen des Verfahrens gewährleistet, daß Hydrazin zu Stickstoff oxidiert wird. Man setzt das Oxidationsmittel in der 1-fachen bis 5-fachen, insbesondere 1,05-fachen bis 2-fachen Menge, die für die Oxidation von Hydrazin zu Stickstoff erforderlich ist, ein.

Üblicherweise setzt man das Oxidationsmittel wenigstens in der für die Oxidation von Hydrazin zu Stickstoff stöchiometrisch erforderlichen Menge oder bis zu einem Überschuß von 2 bis 30, insbesondere 3 bis 10 % der stöchiometrisch erforderlichen Menge.

Setzt man das Oxidationsmittel im Überschuß ein, so kann es günstig sein, nach Beendigung der Umsetzung noch vorhandenes Oxidationsmittel durch Zugabe eines Reduktionsmittel zu beseitigen.

Üblicherweise setzt man als Oxidationsmittel ein Halogen, ein Hypohalogenit, Wasserstoffperoxid, NO₂, N₂O₄, N₂O₃, N₂O₅, ein Nitrit oder eine Mischung derselben ein. Man kann auch Stoffe einsetzen, aus denen sich das eigentliche Oxidationsmittel bildet.

Gut geeignet als Oxidationsmittel sind Cl₂, Br₂, J₂, ClO⁻, BrO⁻, JO⁻ oder Mischungen derselben. Besonders günstig gestaltet sich die Verwendung von Hypohalogenit enthaltenden Halogenbleichlaugen, insbesondere von Hypohalogenit enthaltender Chlorbleichlauge. Man kann aber auch durch Einleiten von Halogen in das basische Reaktionsmedium das Hypohalogenit in situ erzeugen.

Wie eingangs erwähnt, führt man das erfindungsgemäße Verfahren bei einer Temperatur von -10 bis 150, insbesondere -5 bis 110, bevorzugt 0 bis 90°C durch.

Das Verfahren läßt sich, wie zuvor bereits erläutert, flexibel handhaben, indem man zunächst bei niedrigeren Temperaturen, beispielsweise bei -10 bis 60, insbesondere -5 bis 50, bevorzugt 0 bis 40°C die eigentliche Hydrolyse ablaufen läßt und anschließend bei relativ hohen Temperaturen, beispielsweise bei 40 bis 150, insbesondere 50 bis 110, bevorzugt 60 bis 90°C eine weitere Reaktion, beispielsweise eine Austauschreaktion, ablaufen läßt. Als entsprechende Reaktion kommt ein Austausch von Halogen, insbesondere Fluor, gegen Hydroxid in Betracht.
Man kann aber auch die Hydrolyse allein oder die Hydrolyse und die Austauschreaktion von Anfang an bei vergleichsweise hohen Temperaturen durchführen. Die Wahl der Reaktionstemperaturen hängt in gewissen Umfange auch von dem Einsatzstoff ab. Leicht reagierende Einsatzstoffe lassen sich bereits bei vergleichbar niedrigeren Temperaturen umsetzen, während weniger reaktive Einsatzstoffe eine Umsetzung bei hohen Temperaturen erfordern. Auch die Art der Austauschreaktion beeinflußt selbstverständlich die Höhe der Reaktionstemperatur. Handelt es sich um den Austausch einer reaktionsträgen Gruppe oder Restes, so wird man vergleichsweise hohe Temperaturen anwenden.

Man führt die Umsetzung in Anwesenheit oder Abwesenheit eines in Wasser unlöslichen, gegenüber den Reaktionsbedingungen inerten Lösungsmittel durch. Geeignete Lösungsmittel sind, ohne Anspruch auf Vollständigkeit zu erheben, beispielsweise Toluol, o-Xylol, m-Xylol, p-Xylol, Gemische isomerer Xylole, Mesitylen, Ethylbenzol, chlorierte Benzole, wie Chlorbenzol, Dichlorbenzol, Chlortoluol, chlorierte Kohlenwasserstoffe, wie Chloroform, Dichlormethan, Ether, insbesondere Toluol, o-Xylol, m-Xylol, p-Xylol, Gemische isomerer Xylole, Aromaten mit zwei Benzolkernen wie Diphenyl, Diphenylmethan oder Diphenylether, bevorzugt o-Xylol, m-Xylol, p-Xylol, Gemische isomerer Xylole, Diphenylmethan oder Gemische dieser Lösungsmittel. Es bietet sich insbesondere auch an, handelsübliche, alkylierte Benzole und/oder Diphenylether enthaltende Wärmeträgeröle, einzusetzen, die beispielsweise unter der Bezeichnung Dowtherm^{TR}, Diphyl^{TR} oder Santotherm^{TR} im Handel erhältlich sind.

Es kann ebenfalls sinnvoll sein, in Anwesenheit von geringen Mengen inerter, polar aprotischer Lösungsmittel zu arbeiten, wie diese z.B. in den aus der Chlor/Fluor-Austausch-Reaktion erhaltenen Rohprodukten der N'-substituierten N-Amino-tetrafluorphthalimide enthalten sind. Diese Zusätze können möglicherweise aufgrund ihrer Eigenschaft als Lösungsvermittler, zu höheren Reaktionsgeschwindigkeit führen. Somit lassen auch besonders vorteilhaft Rohprodukte, wie sie bei der Synthese, beispielsweise beim Chlor/Fluor-Austausch anfallen, als Startmaterial einsetzen.

Als inerte, polar aprotische Lösungsmittel kommen beispielsweise Sulfolan (Tetramethylensulfon), Tetramethylensulfoxid, Dimethylsulfoxid, Dimethylsulfon, Diphenylsulfoxid, Diphenylsulfon oder Mischungen derselben in Frage. Diese Lösungsmittel sind gegebenenfalls in der als Einsatzprodukt verwendeten Reaktionsmischung in Mengen zwischen etwa 0,1 % und etwa 5 %, bevorzugt zwischen etwa 0,2 % und etwa 2 % enthalten.

Man kann aber auch auf den Zusatz eines Lösungsmittels generell verzichten.

Beabsichtigt man, die entsprechenden substituierten oder unsubstituierten Phthalsäuren herzustellen, so säuert man das anfallende Reaktionsgemisch, beispielsweise durch Zugabe von Mineralsäure, an und trennt die freigesetzte Phthalsäure, beispielsweise durch Filtration oder Extraktion, ab.

Die nachfolgenden Beispiele beschreiben die Erfindung näher.

### Experimenteller Teil

### Beispiel 1

### Herstellung von Tetrachlorphthalsäure

30 g (0,1 Mol) N-Aminotetrachlorphthalimid werden in 150 g Wasser und 32 g (0,8 Mol) Natriumhydroxid eingetragen. Anschließend leitet man unter Rühren bei 20°C über 1 Stunde Chlor ein. Die eindosierte Chlormenge beträgt 17,2 g (0,243 Mol).
Es bildet sich eine feine Suspension, man rührt 5 Stunden bei 30°C nach, vernichtet überschüssiges Chlor bzw. Hypochlorit durch Zugabe von 0,3 g Natriumdithionit und setzt unter Kühlung auf 2°C (Eisbad) Schwefelsäure bis zu einem pH-Wert von 1 zu. Die jetzt besser rührbare Suspension wird abgesaugt und der Filterkuchen zweimal mit je 50 g Wasser gewaschen.

Man erhält nach Trocknen 27,7 g (91 % der Theorie) Tetrachlorphthalsäure als leicht gelbliches Pulver.

### Beispiel 2

### Herstellung eines Gemisches aus Tetrachlorphthalsäure und Benzoesäure

Man arbeitet wie in Beispiel 1 angegeben, setzt jedoch statt N-Aminotetrachlorphthalimid 41 g (0,1 Mol) ca. 98 %iges N'-Benzoyl-N-aminotetrachlorphthalimid und 36 g (0,9 Mol) Natriumhydroxid ein.

Die eindosierte Chlormenge beträgt 18,0 g.
Man arbeitet wie in Beispiel 1 angegeben weiter, vernichtet überschüssiges Chlor bzw. Hypochlorit, säuert an, filtriert und trocknet. Man erhält 39 g eines aus Tetrachlorphthalsäure und Benzoesäure bestehenden, leicht gelblichen Gemisches.

### Beispiel 3

### Herstellung von 3,5,6-Trichlorphthalsäure

50 g (Reingehalt 85 bis 90 %) 3,3',5,5',6,6'-Hexachlorbisphthalimid werden bei 30°C über 1 Stunde in eine Bleichlauge, hergestellt aus 21,3 g (0,3 Mol) Chlor, 40 g (1 Mol) Natriumhydroxid und 150 g Wasser, eingetragen. Man rührt solange nach (5,25 Stunden), bis die HPLC-Chromatographie einen vollständigen Umsatz ausweist. Man vernichtet überschüssiges Chlor bzw. Hypochlorit durch Zugabe von 0,5 g Natriumsulfit bei 20°C und säuert mit technischer Salzsäure (30 %ig) bis zu einem pH-Wert von 1 an.
Die Suspension wird abgesaugt, der Filterkuchen wird mit 100 g Wasser gewaschen.
Man erhält nach Trocknen 22,1 g (82 % der Theorie) 3,5,6-Trichlorphthalsäure als leicht graues, feines Pulver.

### Beispiel 4

### Herstellung von 3,5,6-Trifluorphthalsäure

28 g (Reingehalt 85 bis 90 %) eines rohen braungefärbten 3,3',5,5',6,6'-Hexafluorbisphthalimids, das durch Chlor-Fluor-Austausch aus Hexachlorbisphthalimid hergestellt wurde, werden bei 10°C in eine aus 80 g Wasser und 28 g (85 %iges) Kaliumhydroxid bestehendes Gemisch eingetragen. Anschließend tropft man unter Rühren bei 20°C insgesamt 32 g (0,2 mol) Brom zu und rührt bei dieser Temperatur solange nach (30 Minuten), bis die HPLC-Chromatographie einen vollständigen Umsatz ausweist.
Man vernichtet überschüssiges Brom bzw. Hypobromit durch Zugabe von 3 g Natriumdithionit, säuert bis pH 1 an und extrahiert kontinuierlich (5 Stunden) mit Methyl-tert.-butylether. Die dabei erhaltene organische Phase wird über MgSO₄ getrocknet und das Lösungsmittel im Vakuum entfernt.
Man erhält 13 g (Reingehalt ca. 86 %) eines braunen Öl, entsprechend einer Ausbeute von 80 bis 85 %, bezogen auf eingesetztes Hexafluorbisphthalimid in Reinform.

### Beispiel 5

### Herstellung des Kaliumsalzes von 4-Hydroxy-3,5,6-trifluorphthalsäure

Man legt 85,4 g Wasser und 43,6 g (0,66 Mol) Kaliumhydroxid (85 %ig) vor und trägt bei 5°C über 2 Stunden 25,3 g (0,05 Mol) 86,3 %iges Octafluorbisphthalimid unter Rühren ein. Anschließend leitet man in die Mischung über 30 Minuten unter Rühren bei 5°C (Kühlbad) 7,8 g (0,11 Mol) Chlor ein, wobei die Suspension aufklart und sich in eine Lösung verwandelt. Nach 1 Stunde Nachrühren weist die HPLC-Chromatographie vollständigen Umsatz aus, jedoch liegt noch überschüssiges Chlor bzw. Hypochlorit vor. Man enfernt das Kühlbad und läßt die Temperatur binnen 3 Stunden auf 20°C steigen. Zur Entfernung überschüssigen Chlors bzw. Hypochlorits setzt man 1,8 g Natriumsulfit und anschließend 8,7 g Kaliumhydroxid zu und erhitzt die Mischung unter Rühren 5 Stunden auf 70°C.

Man erhält 187 g einer klaren Lösung, die das Kaliumsalz entsprechend einer Menge von 14,11 g (0,0597 Mol) 4-Hydroxy-3,5,6-trifluorphthalsäure (Ausbeute: 59,7 % der Theorie) enthält.

Diese wäßrige Lösung kann direkt in die Weiterverarbeitung eingesetzt werden, eine Isolierung des Produktes als Salz oder freie Säure ist nicht erforderlich.

### Beispiel 6

### Herstellung des Kaliumsalzes von 4-Hydroxy-3,5,6-trifluorphthalsäure

Man legt 85,4 g Wasser und 43,6 g (0,66 Mol) Kaliumhydroxid (85 %ig) vor und trägt bei 10 bis 15°C über 2 Stunden 25,3 g (0,05 Mol) 86,3 %iges Octafluorbisphthalimid unter Rühren ein. Anschließend setzt man der Mischung über 2 Stunden bei 15 bis 20°C 0,11 Mol Brom in Form einer aus 17,6 g (0,11 Mol) Brom, 21,8 g 85 %iger KOH und 47,7 g Wasser hergestellten Brombleichlauge zu.
Die entstehende braune Lösung wird 1 Stunde nachgerührt und anschließend mit 0,7 g Natriumdithionit versetzt. Man erhitzt danach 6,75 Stunden auf 70°C und erhält 255,7 g einer klaren braunen wäßrigen Lösung, die das Kaliumsalz entsprechend einer Menge von 21,6 g 4-Hydroxy-3,5,6-trifluorphthalsäure (Ausbeute: 91,5 % der Theorie) enthält.
Diese wäßrige Lösung kann direkt in die Weiterverarbeitung eingesetzt werden, eine Isolierung des Produktes als Salz oder freie Säure ist nicht erforderlich.

Man erhält analoge Ergebnisse, wenn man zu Beginn der Umsetzung 6,5 g Kaliumbromid mit dem Wasser und Kaliumhydroxid vorlegt und statt Brombleichlauge eine aus 2,46 l Chlorgas, 21,8 g Kaliumhydroxid und 42,7 g Wasser hergestellte Chlorbleichlauge einsetzt, ansonsten aber wie zuvor beschrieben arbeitet.

### Beispiel 7

### Herstellung von Tetrafluorphthalsäure

Man legt 85,4 g Wasser und 43,6 g (0,66 Mol) 85 %iges Kaliumhydroxid sowie 30 g Xylol vor und trägt bei 0 bis 5°C über 2 Stunden 25,3 g 86,3 %iges Octafluorbisphthalimid unter Rühren ein. Man erhält eine dicke hellbraune Suspension und setzt über 30 Minuten bei 5 bis 7°C 27,6 g (0,11 Mol) Jod zu und tropft gleichzeitig noch 20 g Wasser zu. Am Ende erhält man eine hellbraune, klare Lösung mit einer überstehenden organischen Phase. Man rührt 16 Stunden nach, setzt 3,5 g Natriumsulfit zu und trennt die organische Phase, die verworfen wird, ab. Die wäßrige Phase wird mit 96 %iger Schwefelsäure auf pH = 1 eingestellt und mit Methyl-tert.-butylether extrahiert.

Die organische Phase wird getrocknet und das Lösungsmittel unter Vakuum entfernt.
Man erhält 28,0 g eines braunen, langsam kristallisierenden Rückstandes, der, gemessen mittels kalibrierter HPLC-Chromatographie, 16,2 g (0,068 Mol) Tetrafluorphthalsäure (Ausbeute 68 % der Theorie) enthält.

### Vergleichsbeispiel

### Umsetzung ohne Zusatz eines Oxidationsmittels

Man legt 85,4 g Wasser, 43,6 g 85 %iges Kaliumhydroxid, 30 g Diphenylether und 5 g Xylol vor und trägt bei 15°C über 2 Stunden 25,3 g 86,3 %iges Octafluorbisphthalimid (verteilt auf 8 gleiche Portionen) unter Rühren ein. Man rührt 2 Stunden nach.
Eine Untersuchung (HPLC; ¹H-NMR) zeigt, daß sich lediglich ein einfach ringgeöffnetes Produkt gebildet hat, der Umsatz zu nicht identifizierten Folgeprodukten beträgt < 2 %. Das Kaliumsalz der Tetrafluorphthalsäure bzw. die freie Säure können nicht nachgewiesen werden.
Anschließend erwärmt man auf 23°C und rührt bei dieser Temperatur 18,75 Stunden nach.
Auch nach dieser Zeit liegt die Mischung nach wie vor unverändert vor. Danach erhöht man die Temperatur unter Rühren 2,25 Stunden auf 40 bis 45°C.
Eine Untersuchung zeigt, daß sich nach wie vor lediglich ein einfach ringgeöffnetes Produkt gebildet hat, der Umsatz zu nicht identifizierten Folgeprodukten beträgt etwa 5 %. Das Kaliumsalz der Tetrafluorphthalsäure bzw. die freie Säure können nicht nachgewiesen werden.
Eine Erhöhung der Temperatur über 3 Stunden auf 60°C führt zu einem Produkt, in dem weder das Kaliumsalz der Tetrafluorphthalsäure bzw. die freie Säure noch das Kaliumsalz der 4-Hydroxy-3,5,6-trifluorphthalsäure bzw. die freie Säure nachgewiesen werden können. Es findet zwar ein Umsatz (etwa 40 %) statt, der zu einem Teil zu Zwischenprodukten führt, die nicht weiterreagieren, aber noch - wie das Kontrollexperiment beweist - zum Kaliumsalz der 4-Hydroxy-3,5,6-trifluorphthalsäure umgesetzt werden können.

### Kontrollexperiment (im Anschluß an das Vergleichsbeispiel)

### Fortsetzung des Vergleichsbeispiels mit Zusatz eines Oxidationsmittels

Man setzt dem im Vergleichsbeispiel erhaltenen Reaktionsgemisch über 2,5 Stunden bei 15°C 68,4 g einer 6,7 %igen Chlorbleichlauge und 21 g Wasser unter Rühren zu. Bereits nach Ende der Zugabe ist noch vorhandenes Startmaterial, nämlich das Octafluorbisphthalimid, vollständig zum Kaliumsalz der Tetrafluorphthalsäure umgesetzt.
Man rührt noch 20 Stunden bei dieser Temperatur nach, um die von dem ungewöhnlichen Reaktionsablauf herrührenden Zwischenprodukte noch soweit als möglich umzuwandeln, und setzt anschließend über 5 Stunden bei 70°C zum Kaliumsalz der 4-Hydroxy-3,5,6-trifluorphthalsäure um.

Das Reaktionsgemisch enthält das Kaliumsalz der 4-Hydroxy-3,5,6-trifluorphthalsäure in einer Ausbeute von immerhin noch 79,5 % der Theorie (gemessen mittels HPLC-Chromatographie mit internem Standard).

### Beispiel 8

### Herstellung des Kaliumsalzes von 4-Hydroxy-3,5,6-trifluorphthalsäure

Man legt 247,6 g einer 6,7 %igen Chlorbleichlauge, 17,2 g Wasser, 8,6 g Kaliumhydroxid und 30 g Diphenylether vor und trägt bei 13 bis 21°C über 1,75 Stunden 25,3 g (0,05 Mol) Octafluorbisphthalimid unter Rühren ein. Man rührt 3,5 Stunden nach, wobei nach 3 Stunden 5 g Xylol zugegeben werden. Es fällt ein zwei Phasen enthaltendes Gemisch an. Die wäßrige Phase wird abgetrennt und 2 Stunden unter Rühren auf 70°C erhitzt. Die nach Abkühlen erhaltene wäßrige Lösung enthält das Kaliumsalz entsprechend einer Menge von 13,5 g 4-Hydroxy-3,5,6-trifluorphthalsäure (Ausbeute 57,2 % der Theorie).

### Beispiel 9

### Herstellung des Kaliumsalzes von 4-Hydroxy-3,5,6-trifluorphthalsäure

Man legt 133,1 g Wasser, 65,4 g (0,66 Mol) Kaliumhydroxid, 30 g Dichlortoluol und 10 g eines Gemisches verschiedener aliphatischer Trialkylamine mit 6 bis 14 Kohlenstoffatomen im Alkylrest (Hostarex 327; Handelsprodukt der Hoechst AG) vor und trägt bei 5 bis 10°C über 1 Stunde 25,3 g (0,05 Mol) 86,3 %iges Octafluorbisphthalimid ein. Anschließend gibt man unter gutem Rühren über 5 Stunden bei 10 bis 12°C 17,6 g (0,11 Mol) Brom zu, rührt eine Stunde nach und versetzt danach mit 0,3 g Natriumdithionit. Anschließend erhitzt man 3 Stunden unter Rühren auf 80°C und erhält ein aus 2 Phasen bestehendes Gemisch. Die untere Phase entspricht 279,9 g einer klaren, braunen wäßrigen Phase, die das Kaliumsalz entsprechend einer Menge von 20,0 g 4-Hydroxy-3,5,6-trifluorphthalsäure (Ausbeute: 84,7 % der Theorie) enthält.
Das aus 2 Phasen bestehende Gemisch kann direkt, ohne Phasentrennung und ohne Isolierung eines Produktes, in die Weiterverarbeitung eingesetzt werden.

## Patentansprüche

1. Verfahren zur Herstellung von Salzen substituierter oder unsubstituierter Phthalsäuren, **dadurch gekennzeichnet, daß** man eine Verbindung der allgemeinen Formel 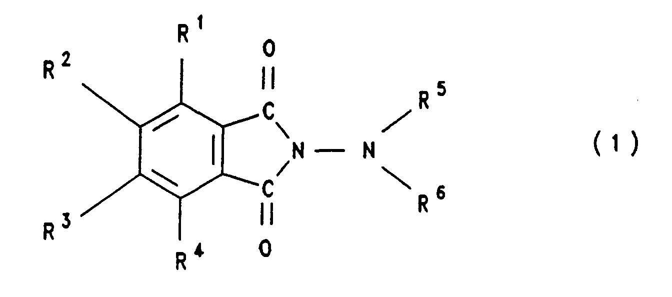 worin R¹, R², R³ und R⁴ gleich oder verschieden sind und für H, F, Cl, Br, CF₃, OH, für einen Alkoxy- oder Alkylrest mit jeweils 1 bis 4 Kohlenstoffatomen oder einen Rest -NR⁷R⁸ stehen, in welchem R⁷ und R⁸ gleich oder verschieden sind und für H, einen Alkylrest mit 1 bis 4 Kohlenstoffatomen oder einen Phenylrest stehen, R⁵ und R⁶ gleich oder verschieden sind und für H, eine -CO-Alkylgruppe mit 1 bis 6 Kohlenstoffatomen im Alkylrest oder eine Benzoylgruppe stehen, oder R⁵ und R⁶ zusammen einen Rest der allgemeinen Formel 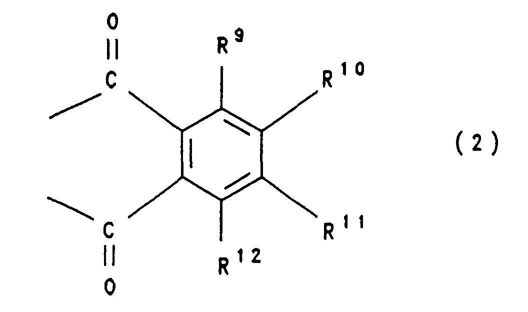 bilden, worin R⁹, R¹⁰, R¹¹ und R¹² gleich oder verschieden sind und für H, F, Cl, Br, CF₃, OH, für einen Alkoxy- oder Alkylrest mit jeweils 1 bis 4 Kohlenstoffatomen oder einen Rest -NR⁷R⁸ stehen, in welchem R⁷ und R⁸ gleich oder verschieden sind und für H, einen Alkylrest mit 1 bis 4 Kohlenstoffatomen oder einen Phenylrest stehen, mit Wasser, 2 bis 30 Äquivalenten einer in Wasser löslichen Base je Mol abzuspaltender Phthalsäuresalze und einem Oxidationsmittel bei einer Temperatur von -10 bis 150°C in Anwesenheit oder Abwesenheit eines in Wasser unlöslichen, gegenüber den Reaktionsbedingungen inerten Lösungsmittels umsetzt, wobei die in Wasser lösliche Base und das Oxidationsmittel gleichzeitig eingesetzt werden.

2. Verfahren nach Anspruch 1, **dadurch gekennzeichnet, daß** man eine Verbindung der Formel (1) einsetzt, worin R¹, R², R³ und R⁴ gleich oder verschieden sind und für H, F, Cl, OH oder für einen Alkoxyrest mit 1 bis 4 Kohlenstoffatomen stehen.

3. Verfahren nach Anspruch 1 oder 2, **dadurch gekennzeichnet, daß** man eine Verbindung der Formel (1) einsetzt, worin R¹, R², R³ und R⁴ gleich oder verschieden sind und für F, OH oder einen Alkoxyrest mit 1 bis 4 Kohlenstoffatomen stehen.

4. Verfahren nach einem oder mehreren der Ansprüche 1 bis 3, **dadurch gekennzeichnet, daß** man eine Verbindung der Formel (1) einsetzt, worin R⁵ und R⁶ einen Rest (2) bilden, R⁹, R¹⁰, R¹¹ und R¹² gleich oder verschieden sind und für H, F, Cl, OH oder für einen Alkoxyrest mit 1 bis 4 Kohlenstoffatomen stehen.

5. Verfahren nach einem oder mehreren der Ansprüche 1 bis 4, **dadurch gekennzeichnet, daß** man eine Verbindung der Formel (1) einsetzt, worin R⁵ und R⁶ einen Rest (2) bilden, R⁹, R¹⁰, R¹¹ und R¹² gleich oder verschieden sind und für F, OH oder einen Alkoxyrest mit 1 bis 4 Kohlenstoffatomen stehen.

6. Verfahren nach einem oder mehreren der Ansprüche 1 bis 5, **dadurch gekennzeichnet, daß** man eine Verbindung der Formel (3) 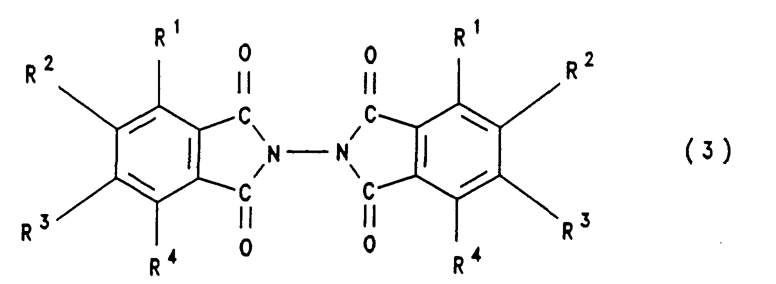 einsetzt, worin R¹, R², R³ und R⁴ die in Anspruch 1 angegebene Bedeutung haben.

7. Verfahren nach einem oder mehreren der Ansprüche 1 bis 6, **dadurch gekennzeichnet, daß** man Oktafluorbisphthalimid 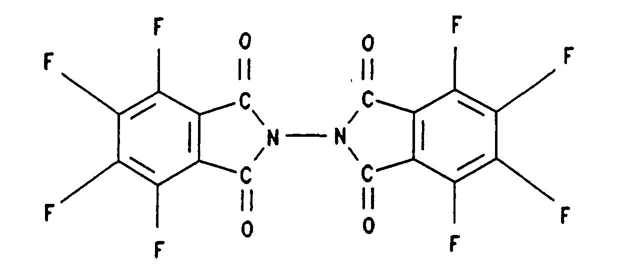 einsetzt.

8. Verfahren nach einem oder mehreren der Ansprüche 1 bis 7, **dadurch gekennzeichnet, daß** man 2 bis 3000, insbesondere 10 bis 500 bevorzugt 30 bis 200 Mol Wasser je Mol abzuspaltender Phthalsäuresalze einsetzt.

9. Verfahren nach einem oder mehreren der Ansprüche 1 bis 8, **dadurch gekennzeichnet, daß** man 3 bis 15, insbesondere 4 bis 10 Äquivalente der in Wasser löslichen Base je Mol abzuspaltender Phthalsäuresalze einsetzt.

10. Verfahren nach einem oder mehreren der Ansprüche 1 bis 9, **dadurch gekennzeichnet, daß** man als in Wasser lösliche Base ein Alkalioxid, Alkalihydroxid, Erdalkalioxid oder Erdalkalihydroxid oder ein Gemisch derselben einsetzt.

11. Verfahren nach einem oder mehreren der Ansprüche 1 bis 10, **dadurch gekennzeichnet, daß** man als in Wasser lösliche Base Natriumhydroxid oder Kaliumhydroxid einsetzt.

12. Verfahren nach einem oder mehreren der Ansprüche 1 bis 11, **dadurch gekennzeichnet, daß** man das Oxidationsmittel in der 1-fachen bis 5-fachen Menge, die für die Oxidation von Hydrazin zu Stickstoff erforderlich ist, zusetzt.

13. Verfahren nach einem oder mehreren der Ansprüche 1 bis 12, **dadurch gekennzeichnet, daß** man das Oxidationsmittel in der 1,05-fachen bis 2-fachen Menge, die für die Oxidation von Hydrazin zu Stickstoff erforderlich ist, zusetzt.

14. Verfahren nach einem oder mehreren der Ansprüche 1 bis 13, **dadurch gekennzeichnet, daß** man als Oxidationsmittel ein Halogen, ein Hypohalogenit, Wasserstoffperoxid, NO₂, N₂O₄, N₃O₃, N₂O₅, ein Nitrit oder eine Mischung derselben einsetzt.

15. Verfahren nach einem oder mehreren der Ansprüche 1 bis 14, **dadurch gekennzeichnet, daß** man als Oxidationsmittel Cl₂, Br₂, J₂, ClO⁻, BrO⁻, JO⁻ oder Mischungen derselben einsetzt.

16. Verfahren nach einem oder mehreren der Ansprüche 1 bis 17, **dadurch gekennzeichnet, daß** man die Umsetzung bei -5 bis 110, insbesondere 0 bis 90°C durchführt.

## Claims

1. A process for the preparation of salts of substituted or unsubstituted phthalic acids, which comprises reacting a compound of the formula 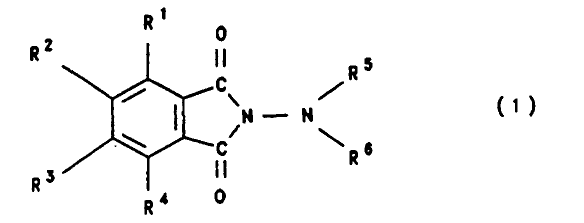 in which R¹, R², R³ and R⁴ are identical or different and are H, F, Cl, Br, CF₃, OH, an alkoxy or alkyl radical in each case having 1 to 4 carbon atoms, or a radical -NR⁷R⁸, in which R⁷ and R⁸ are identical or different and are H, an alkyl radical having 1 to 4 carbon atoms, or a phenyl radical, R⁵ and R⁶ are identical or different and are H, a -CO-alkyl group having 1 to 6 carbon atoms in the alkyl radical, or a benzoyl group, or R⁵ and R⁶ together form a radical of the formula 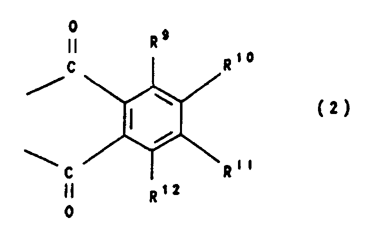 in which R⁹, R¹⁰, R¹¹ and R¹² are identical or different and are H, F, Cl, Br, CF₃, OH, an alkoxy or alkyl radical in each case having 1 to 4 carbon atoms, or a radical -NR⁷R⁸, in which R⁷ and R⁸ are identical or different and are H, an alkyl radical having 1 to 4 carbon atoms, or a phenyl radical, with water, 2 to 30 equivalents of a water-soluble base per mole of phthalic salts to be released and an oxidizing agent at a temperature of -10 to 150°C in the presence or absence of a water-insoluble solvent inert under the reaction conditions wherein the water-soluble base and the oxidizing agent are introduced simultaneously.

2. The process as claimed in claim 1, wherein a compound of the formula (1) is used in which R¹, R², R³ and R⁴ are identical or different and are H, F, Cl, OH or an alkoxy radical having 1 to 4 carbon atoms.

3. The process as claimed in claim 1 or 2, wherein a compound of the formula (1) is used in which R¹, R², R³ and R⁴ are identical or different and are F, OH or an alkoxy radical having 1 to 4 carbon atoms.

4. The process as claimed in one or more of claims 1 to 3, wherein a compound of the formula (1) is used in which R⁵ and R⁶ form a radical (2), R⁹, R¹⁰, R¹¹ and R¹² are identical or different and are H, F, Cl, OH or an alkoxy radical having 1 to 4 carbon atoms.

5. The process as claimed in one or more of claims 1 to 4, wherein a compound of the formula (1) is used in which R⁵ and R⁶ form a radical (2), R⁹, R¹⁰, R¹¹ and R¹² are identical or different and are F, OH or an alkoxy radical having 1 to 4 carbon atoms.

6. The process as claimed in one or more of claims 1 to 5, wherein a compound of the formula (3) 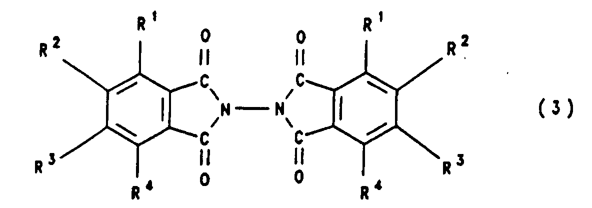 in which R¹, R², R³ and R⁴ have the meanings given above is used.

7. The process as claimed in one or more of claims 1 to 6, wherein octafluorobisphthalimide 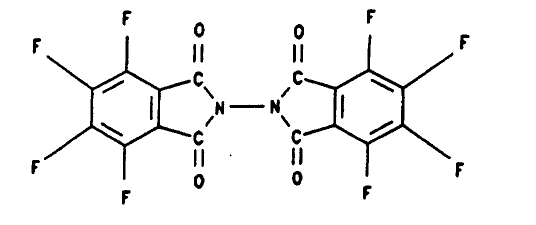 is used.

8. The process as claimed in one or more of claims 1 to 7, wherein 2 to 3000, in particular 10 to 500, preferably 30 to 200, mol of water are used per mole of phthalic salts to be released.

9. The process as claimed in one or more of claims 1 to 8, wherein 3 to 15, in particular 4 to 10, equivalents of the water-soluble base are used per mole of phthalic salts to be released.

10. The process as claimed in one or more of claims 1 to 9, wherein the water-soluble base used is an alkali metal oxide, alkali metal hydroxide, alkaline earth metal oxide or alkaline earth metal hydroxide or a mixture of the same.

11. The process as claimed in one or more of claims 1 to 10, wherein the water-soluble base used is sodium hydroxide or potassium hydroxide.

12. The process as claimed in one or more of claims 1 to 11, wherein the oxidizing agent is added at 1 to 5 times the amount which is required for the oxidation of hydrazine to nitrogen.

13. The process as claimed in one or more of claims 1 to 12, wherein the oxidizing agent is added at 1.05 to 2 times the amount which is required for the oxidation of hydrazine to nitrogen.

14. The process as claimed in one or more of claims 1 to 13, wherein the oxidizing agent used is a halogen, a hypohalite, hydrogen peroxide, NO₂, N₂O₄, N₂O₃, N₂O₅, a nitrite or a mixture of the same.

15. The process as claimed in one or more of claims 1 to 14, wherein the oxidizing agent used is Cl₂, Br₂, I₂, ClO⁻, BrO⁻, IO⁻ or mixtures of the same.

16. The process as claimed in one or more of claims 1 to 15, wherein the reaction is carried out at -5 to 110, in particular 0 to 90, °C.

## Revendications

1. Procédé de préparation de sels d'acides phtaliques substitués ou non substitués, **caractérisé en ce qu'**on fait réagir un composé de formule générale 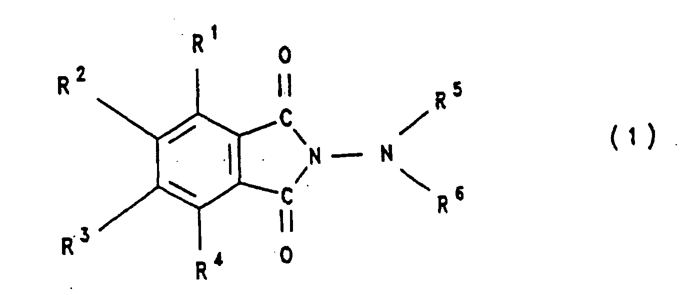 dans laquelle R¹, R², R³ et R⁴ sont identiques ou différents et représentent chacun H, F, Cl, Br, CF₃, OH, un radical alcoxy ou alkyle ayant chacun de 1 à 4 atomes de carbone, ou un radical -NR⁷R⁸ où R⁷ et R⁸ sont identiques ou différents et représentent chacun H, un radical alkyle ayant de 1 à 4 atomes de carbone ou un radical phényle, R⁵ et R⁶ sont identiques ou différents et représentent chacun H, un groupe -CO-alkyle ayant de 1 à 6 atomes de carbone dans le fragment alkyle ou un groupe benzoyle, ou encore R⁵ et R⁶ forment ensemble un radical de formule générale 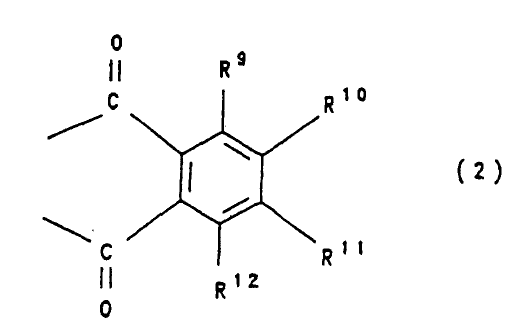 dans laquelle R⁹, R¹⁰, R¹¹ et R¹² sont identiques ou différents et représentent chacun H, F, Cl, Br, CF₃, OH, un radical alcoxy ou alkyle ayant chacun de 1 à 4 atomes de carbone ou un radical -NR⁷R⁸ dans lequel R⁷ et R⁸ sont identiques ou différents et représentent chacun H, un radical alkyle ayant de 1 à 4 atomes de carbone ou un radical phényle, avec de l'eau, 2 à 30 équivalents d'une base soluble dans l'eau par mole de sels de l'acide phtalique à dissocier, et un oxydant, à une température de -10 à 150°C, en présence ou en l'absence d'un solvant insoluble dans l'eau et inerte dans les conditions de la réaction, la base soluble dans l'eau et l'oxydant étant utilisés simultanément.

2. Procédé selon la revendication 1, **caractérisé en ce qu'**on utilise un composé de formule (1) dans laquelle R¹, R², R³ et R⁴ sont identiques ou différents et représentent chacun H, F, Cl, OH, ou un radical alcoxy ayant de 1 à 4 atomes de carbone.

3. Procédé selon la revendication 1 ou 2, **caractérisé en ce qu'**on utilise un composé de formule (1) dans laquelle R¹, R², R³ et R⁴ sont identiques ou différents et représentent chacun F, OH ou un radical alcoxy ayant de 1 à 4 atomes de carbone.

4. Procédé selon l'une ou plusieurs des revendications 1 à 3, **caractérisé en ce qu'**on utilise un composé de formule (1) dans laquelle R⁵ et R⁶ forment un radical (2), R⁹, R¹⁰, R¹¹ et R¹² sont identiques ou différents et représentent chacun H, F, Cl, OH ou un radical alcoxy ayant de 1 à 4 atomes de carbone.

5. Procédé selon l'une ou plusieurs des revendications 1 à 4, **caractérisé en ce qu'**on utilise un composé de formule (1) dans laquelle R⁵ et R⁶ forment un radical (2), R⁹, R¹⁰, R¹¹ et R¹² sont identiques ou différents et représentent chacun F, OH ou un radical alcoxy ayant de 1 à 4 atomes de carbone.

6. Procédé selon l'une ou plusieurs des revendications 1 à 5, **caractérisé en ce qu'**on utilise un composé de formule (3) 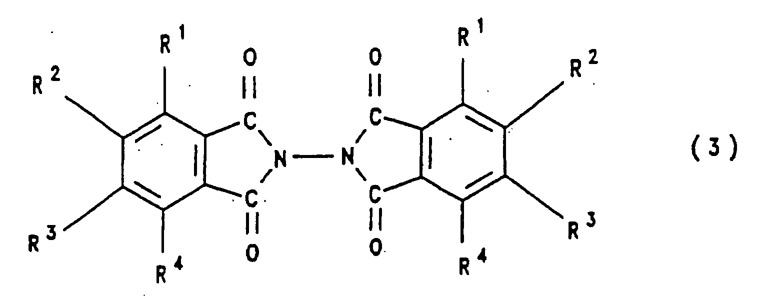 dans laquelle R¹, R², R³ et R⁴ ont les significations données dans la revendication 1.

7. Procédé selon l'une ou plusieurs des revendications 1 à 6, **caractérisé en ce qu'**on utilise de l'octafluorobisphtalimide

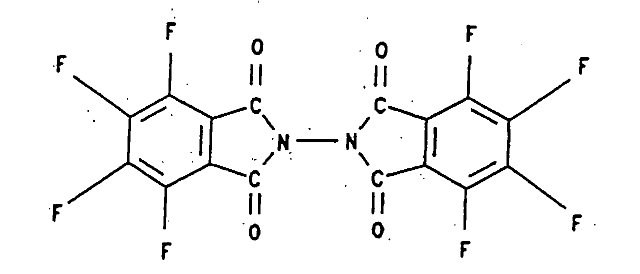

8. Procédé selon l'une ou plusieurs des revendications 1 à 7, **caractérisé en ce qu'**on utilise de 2 à 3000, en particulier de 10 à 500 et de préférence de 30 à 200 moles d'eau par mole de sels d'acide phtalique à dissocier.

9. Procédé selon l'une ou plusieurs des revendications 1 à 8, **caractérisé en ce qu'**on utilise de 3 à 15 et en particulier de 4 à 10 équivalents de la base soluble dans l'eau par mole de sels de l'acide phtalique à dissocier.

10. Procédé selon l'une ou plusieurs des revendications 1 à 9, **caractérisé en ce qu'**on utilise en tant que base soluble dans l'eau l'oxyde d'un métal alcalin, l'hydroxyde d'un métal alcalin, l'oxyde d'un métal-alcalino-terreux ou l'hydroxyde d'un métal alcalino-terreux ou un mélange de ceux-ci.

11. Procédé selon l'une ou plusieurs des revendications 1 à 10, **caractérisé en ce qu'**on utilise en tant que base soluble dans l'eau l'hydroxyde de sodium ou l'hydroxyde de potassium.

12. Procédé selon l'une ou plusieurs des revendications 1 à 11, **caractérisé en ce qu'**on ajoute l'oxydant en une quantité 1 à 5 fois supérieure à celle qui est nécessaire à l'oxydation de l'hydrazine en azote.

13. Procédé selon l'une ou plusieurs des revendications 1 à 12, **caractérisé en ce qu'**on ajoute l'oxydant en une quantité 1,05 à 2 fois supérieure à celle qui est nécessaire à l'oxydation de l'hydrazine en azote.

14. Procédé selon l'une ou plusieurs des revendications 1 à 13, **caractérisé en ce qu'**on utilise en tant qu'oxydant un halogène, un hypohalogénite, du peroxyde d'hydrogène, du NO₂, du N₂O₄, du N₂O₃, du N₂O₅, un nitrite ou un mélange de ceux-ci.

15. Procédé selon l'une ou plusieurs des revendications 1 à 14, **caractérisé en ce qu'**on utilise en tant qu'oxydant du Cl₂, du Br₂, de l'I₂, du ClO⁻, du BrO⁻, de l'IO⁻ ou des mélanges de ceux-ci.

16. Procédé selon l'une ou plusieurs des revendications 1 à 17, **caractérisé en ce qu'**on met en oeuvre la réaction à une température de -5 à 110 et en particulier de 0 à 90°C.
